# EUROPÄISCHE PATENTSCHRIFT

(11) **EP 3 206 873 B1**
(45) Veröffentlichungstag und Bekanntmachung des Hinweises auf die Patenterteilung: **12.12.2018**
(21) Anmeldenummer: 15778298.8
(22) Anmeldetag: 09.10.2015
(51) Int. Cl.: B32B 37/00, A61K 9/70, B32B 37/26

(54) **UMKASCHIERVORRICHTUNG**
LAMINATING DEVICE
DISPOSITIF DE CHEVAUCHEMENT

(30) Priorität: 14.10.2014 EP 14188784
(43) Veröffentlichungstag der Anmeldung: 23.08.2017
(73) Patentinhaber: Bayer Pharma Aktiengesellschaft, 13353 Berlin (DE)
(72) Erfinder: GRADER, Ludwig, 56626 Andernach (DE); KILLER, Roland, 83626 Valley (DE)
(74) Vertreter: Meinken Zehetner Patentanwälte
(86) Internationale Anmeldenummer: PCT/EP2015/073327
(87) Internationale Veröffentlichungsnummer: WO 2016/058911

(56) Entgegenhaltungen:
- WO-A1-2013/014893
- DE-A1- 10 056 855
- DE-C1- 19 837 764
- US-A1- 2005 211 369

## Beschreibung

Die vorliegende Erfindung bezieht sich auf die Übertragung einer zugempfindlichen Folie von einem ersten bandförmigen Träger auf einen zweiten bandförmigen Träger im Allgemeinen und im Besonderen auf ein Umkaschieren eines zugempfindlichen Rückschichtfolienbandes von einem Trägerband auf ein bandförmiges Laminat bzw. auf ein Umkaschieren eines von einem zugempfindlichen Rückschichtfolienband und einer an diesem haftenden Klebeschicht gebildeten Laminats von einem Trägerband auf ein von einem oder mehreren Streifen gebildetes Schutzfolienband. Ein solches Verfahren ist aus DE-A-10 56 855 bekannt.

Das Umkaschieren von zugempfindlichen Folien von einem ersten bandförmigen Träger auf einen zweiten bandförmigen Träger ist beispielsweise bei der Herstellung von hochelastischen transdermalen therapeutischen Systemen von Bedeutung, die, um im Applikationsfall einen dauerhaften vollflächigen Hautkontakt zu gewährleisten, allen Verformungen der von ihnen überdeckten Hautoberfläche folgen können müssen.

Transdermale therapeutische Systeme (TTS), häufig auch kurz als TTS-Pflaster bezeichnet, werden in der Regel auf der Haut eines Patienten angebracht, so dass der bzw. die in dem TTS enthaltenen Wirkstoffe aus dem transdermalen therapeutischen System in die Haut und hierüber in die Blutgefäße des Patienten gelangen können.

TTS-Pflaster umfassen gewöhnlich ein Wirkstoffdepot, eine wirkstoffundurchlässige Rückschicht zum Abdecken der nicht zum Kontakt mit der Haut vorgesehenen Seite des Wirkstoffdepots und eine Schutzfolie zum Abdecken der zum Kontakt mit der Haut vorgesehenen Applikationsseite des Wirkstoffdepots. Die Schutzfolie ist gewöhnlich an einer selbstklebend ausgeführten Oberfläche des Wirkstoffdepots befestigt. Unter selbstklebend ist hierbei das Vermögen zu verstehen, bei Kontakt mit einer Oberfläche eines Gegenstands an dieser anzuhaften, wobei eine solche adhäsive Verbindung ohne substantielle Beeinträchtigung der beiden Adhäsionspartner wieder gelöst werden kann. Der Begriff selbstklebend wird in dieser Schrift somit synonym zum Begriff haftklebend verwendet. Die Schutzfolie dient einerseits dem Schutz der Applikationsseite vor Verschmutzung und anderen Außeneinwirkungen, andererseits aber auch zum Schutz der Umgebung und insbesondere von Personen vor einer eventuellen Kontamination durch den bzw. die in dem TTS-Pflaster enthaltenen Wirkstoff(e). Die Schutzfolie wird vor der Applikation des Systems entfernt.

Gegenwärtig werden zwei Grundtypen transdermaler therapeutischer Systeme verwendet, Matrixsysteme und Reservoirsysteme. Reservoirsysteme enthalten den bzw. die Wirkstoff(e) in einem flüssigen, halbfesten oder festen Reservoir, wobei die Wirkstoffabgabe üblicherweise mittels einer Membran reguliert wird. Bei Matrixsystemen ist der bzw. die Wirkstoff(e) in einer Polymermatrix eingebettet vorgehalten. Die Wirkstoffabgabe regelt sich über das Konzentrationsgefälle des Wirkstoffs zur Haut.

Zur Herstellung transdermaler therapeutischer Matrixsysteme wird in der Regel eine den bzw. die Wirkstoff(e) enthaltende selbstklebende Polymermatrix bahnförmig auf ein Trägerfolienband aufgebracht. Unter Bahn ist in dieser Schrift ein Gebilde mit begrenzter Breite und nicht näher spezifizierter Länge zu verstehen. Unter einem Band ist ein dünner, selbstragender, einschichtiger oder mehrschichtiger Körper von bestimmter Breite aber unbestimmter Länge zu verstehen, wobei die Länge eines Bandes dessen Breite üblicherweise um ein Vielfaches übersteigt.

Die auch als Wirkstoffdepot bezeichnete wirkstoffhaltige Polymermatrix kann dabei schmaler als das Trägerfolienband sein und auch in mehreren zueinander beabstandeten Bahnen auf die Trägerfolie aufgebracht werden. Unter "beabstandeten Bahnen" werden im Sinne der Erfindung einzelne Bahnen verstanden, die durch einen Zwischenraum voneinander getrennt sind. Statt eines Wirkstoffs kann eine Polymermatrix auch mehrere Wirkstoffe enthalten und z. B. zur Steuerung der Wirkstofffreisetzung gegebenenfalls auch mehrschichtig aufgebaut sein, wobei unterschiedliche Schichten der Polymermatrix unterschiedliche Wirkstoffe bzw. Wirkstoffzusammensetzungen enthalten und manche auch frei von Wirkstoffen sein können.

Auf der der Trägerfolie abgewandten Oberseite des Wirkstoffdepots wird oftmals ein wirkstoffundurchlässiges Rückschichtband aufgebracht, das hierzu von einem sie tragenden Stützfolienband abgezogen werden muss.

Bei einigen Herstellungsverfahren weist das ein Wirkstoffdepot tragende Trägerfolienband nicht die für eine TTS-Schutzfolie erforderlichen Eigenschaften auf. Die Eigenschaften einer TTS-Schutzfolie richten sich nach dem jeweiligen Anwendungs- bzw. Einsatzzweck eines TTS. Die Schutzfolie kann beispielsweise überlappend ausgeführt werden, um einem Austritt einer kaltfließenden Matrix entgegenzuwirken. Ferner ist die Verwendung von mehrteiligen Schutzfolien mit unterschiedlicher Flexibilität der Einzelfolien bekannt. Auch geprägte Schutzfolien werden in der Praxis eingesetzt. Die vielfältigen Eigenschaften von Trägerfolie richten sich nach dem Herstellungsprozess. Wenn die Trägerfolie nicht als Schutzfolie verwendet werden kann, dann muss sie durch eine Schutzfolie ersetzt werden.

In diesen Fällen muss das bandförmige Laminat aus Wirkstoffdepot und daran haftender Rückschicht von der Trägerfolie abgelöst und zur Bildung eines TTS-Laminats auf ein Schutzfolienband übertragen werden. Das Schutzfolienband kann von einem oder von mehreren streifenförmigen Bändern gebildet sein. Bei Schutzfolienbändern, die aus mehreren streifenförmigen Bändern gebildet sind, können die Streifen entweder direkt aneinandergrenzend oder einander überlappend angeordnet sein.

Zur Vereinzelung der transdermalen therapeutischen Systeme werden aus dem TTS-Laminat üblicherweise den TTS-Pflastern entsprechende Bereiche ausgeschnitten bzw. ausgestanzt und verpackt.

Zum Umkaschieren eines als Folie bzw. Laminat ausgebildeten Materialbandes von einem Trägerband auf ein anderes muss das Materialband zunächst von dem Trägerband abgezogen und dann auf ein anderes Trägerband aufkaschiert werden. Unter Kaschieren ist hierbei das Verbinden von zumindest zwei Materiallagen bzw. Materialbändern zu verstehen, wobei die Verbindung mittels Klebstoff oder ohne Klebstoff, beispielsweise unter Anwendung von Wärme, Druck oder Vakuum erfolgen kann. Ein Vorgang zum Kaschieren eines Materialbandes mit einem anderen Materialband wird als Aufkaschieren bezeichnet. Ein Vorgang zum Abziehen bzw. Ablösen eines aufkaschierten Materialbandes von einem Trägerband wird im Folgenden auch als Dekaschieren bezeichnet.

Das Dekaschieren erfolgt üblicherweise durch Auftrennen eines Laminatbandes in Teilbänder, die jeweils Teil des ursprünglichen Laminatbandes waren. Beim Dekaschieren wird zumindest eines der Teilbänder an einer Rolle oder Walze oder an einer Kante (manchmal auch als Spenderkante bezeichnet) umgelenkt. Vorrichtungen, die eine Umlenkung eines Material- oder Trägerbandes an einer sogenannten Spenderkante ermöglichen, sind z. B. aus der Patentschrift DE 44 06 976 C1 bekannt.

Bei einem Dekaschieren mittels einer durch Walzen bzw. Rollen oder Spenderkanten bewirkten Umlenkung des abzuziehenden Teils, des anderen Teils oder beider Teile eines Laminatbandes werden auf die Teilbänder Zugkräfte ausgeübt, die, um ein zuverlässiges Auftrennen des Laminats zu gewährleisten, erfahrungsgemäß mindestens doppelt so hoch sein müssen wie die Adhäsionskräfte, welche die zu trennenden Laminatbandschichten verbinden.

Handelt es sich bei der umzukaschierenden Laminatbandschicht um eine zugempfindliche Folie, d.h. eine Folie, die bereits bei geringen Zugbeanspruchungen Formveränderungen erfährt, führen solche Zugkräfte zu einer starken Dehnung der Folie. Wird die so gedehnte Folie zur Bildung eines zweiten Laminats auf ein zweites Trägerband aufgebracht, dann treten in diesem Laminat aufgrund der Dehnung Spannungen auf.

Bei Verwendung eines flexiblen, jedoch nicht oder nur geringfügig elastischen, zweiten bandförmigen Trägers wird die Dehnung der zugempfindlichen Folie beim Aufbringen eingefroren, d. h. die Dehnung der Folie bleibt im Laminat erhalten. Unter flexibel ist hierbei die Eigenschaft eines Materials zu verstehen, sich infolge einer senkrecht zu einer seiner Oberflächen einwirkenden Kraft zu verbiegen. Die bei entsprechenden Laminatbändern zwischen der gedehnten Folienschicht und der flexiblen Trägerschicht auftretenden Spannungen führen zum als Rolleffekt bekannten Einrollen von aus dem Laminatband hergestellten transdermalen therapeutischen Systemen, die deren Handhabung erschwert. Ist der zweite bandförmige Träger zudem elastisch, dann können diese Spannungen auch zu einer Schrumpf genannten Verkürzung von aus dem Laminatband hergestellten transdermalen therapeutischen Systemen und damit zu einer Unbestimmtheit der Wirkstoffflächenkonzentration derartiger TTS-Pflaster führen. Ferner neigen solche Pflaster häufig zur Faltenbildung, die das erforderliche ganzflächige Aufbringen der TTS-Pflaster auf der Haut eines Patienten erschwert und bisweilen unmöglich macht.

Es ist daher Aufgabe der vorliegenden Erfindung, eine Vorrichtung zur Verfügung zu stellen, die ein Umkaschieren einer zugempfindlichen Folie von einem ersten bandförmigen Träger auf einen zweiten bandförmigen Träger mit einer so geringen Dehnung der Folie ermöglicht, dass der zweite Träger durch die auf ihn aufkaschierte Folie nicht verformt wird.

Eine entsprechende Vorrichtung ist durch die Kombination der in Anspruch 1 angegebenen Merkmale definiert. Bevorzugte Weiterbildungen der Vorrichtung sind jeweils Gegenstand von Unteransprüchen.

Ausführungsformen einer solchen Vorrichtung zum Umkaschieren zugempfindlicher Folien von einem ersten Träger auf einen zweiten Träger weisen eine erste Transporteinrichtung, eine zweite Transporteinrichtung und eine Aufkaschiereinrichtung auf. Die erste Transporteinrichtung ist hierbei zum Transport eines ersten Laminatbandes, das von einem ersten bandförmigen Träger und einer darauf aufkaschierten zugempfindlichen Folie gebildet ist, von einer Laminatausgabeeinrichtung zu einer Foliendekaschierstelle und zum Weitertransport des an der Foliendekaschierstelle von der zugempfindlichen Folie abgetrennten Trägerbandes von der Foliendekaschierstelle zu einer Trägerbandaufnahmeeinrichtung ausgebildet. Die zweite Transporteinrichtung ist des Weiteren zum Transport eines zweiten bandförmigen Trägers zu einer Aufkaschiereinrichtung, die zum Aufkaschieren einer an der Foliendekaschierstelle dekaschierten zugempfindlichen Folie auf den zweiten Träger zur Bildung eines zweiten Laminatbandes ausgebildet ist, und zum Weitertransport des zweiten Laminatbandes von der Aufkaschiereinrichtung zu einer Laminatbandaufnahmeeinrichtung ausgebildet. Ferner sind erste Transporteinrichtung und Aufkaschiereinrichtung relativ zueinander so angeordnet, dass die zugempfindliche Folie an der Foliendekaschierstelle in zur Laufrichtung des ersten Laminatbandes an dieser Stelle entgegengesetzten Richtung abgezogen wird.

Eine entsprechende Vorrichtung minimiert die zum Ablösen der zugempfindlichen Folie von dem ersten Träger erforderlichen Zugkräfte, da sich durch die Umlenkung der Folie die Trennkräfte auf die Ablöse- bzw. Dekaschierstelle konzentrieren.

Es wird darauf hingewiesen, dass die in dieser Beschreibung und den Ansprüchen zur Aufzählung von Merkmalen verwendeten Begriffe "umfassen", "aufweisen", "beinhalten", "enthalten" und "mit", sowie deren grammatikalischen Abwandlungen, generell als nichtabschließende Aufzählung von Merkmalen, wie z. B. Verfahrensschritten, Einrichtungen, Bereichen, Größen und dergleichen aufzufassen sind, und in keiner Weise das Vorhandensein anderer oder zusätzlicher Merkmale oder Gruppierungen von anderen oder zusätzlichen Merkmalen ausschließen.

Um Schwankungen von auf die zugempfindliche Folie einwirkenden Zugkräften zu vermeiden, weisen bevorzugte Ausführungsformen von wie zuvor angegebenen Umkaschiervorrichtungen ferner eine Einrichtung zur Lagestabilisierung der Foliendekaschierstelle auf. Zweckmäßige Ausgestaltungen solcher Einrichtungen weisen zumindest einen Sensor auf, der zur Ausgabe von zumindest einem für eine aktuelle Lage der Foliendekaschierstelle repräsentativen Sensorsignal ausgebildet ist. Um eine geregelte Lagestabilisierung der Foliendekaschierstelle zu erreichen, weisen vorteilhafte Ausführungsformen ferner eine Steuerung auf, die so zur Steuerung von erster Transporteinrichtung und zweiter Transporteinrichtung in Abhängigkeit des Sensorsignals ausgebildet ist, dass sich die Lage der Foliendekaschierstelle relativ zu erster Transporteinrichtung und Aufkaschiereinrichtung nicht bzw. nicht in technisch relevanter Weise verändert. Es sei in diesem Zusammenhang darauf hingewiesen, dass abweichend vom deutschen Sprachgebrauch in dieser Schrift nicht zwischen den Begriffen Steuern und Regeln unterschieden wird. Vielmehr werden beide Begriffe synonym verwendet, d.h. der Begriff Steuern kann ebenso eine Rückführung einer Regelgröße bzw. deren Messwerts umfassen, wie sich der Begriff Regeln auf eine einfache Steuerkette beziehen kann. Dies betrifft auch grammatikalische Abwandlungen dieser Begriffe.

Zum Umkaschieren von als Laminat ausgebildeten zugempfindlichen Folien, die an einer Folienseite eine haftklebende Schicht aufweisen, sind zweckmäßige Ausgestaltungen von wie zuvor angegebenen Umkaschiervorrichtungen so ausgebildet, dass die zugempfindliche Folie direkt, d.h. ohne zwischendurch ein Führungselement mit der selbst- bzw. haftklebenden Seite zu berühren, von der Foliendekaschierstelle zur Aufkaschiereinrichtung geführt wird.

Zum Umkaschieren von zugempfindlichen Folien, die keine haftklebenden Oberflächen aufweisen, weisen Ausgestaltungen von wie zuvor angegebenen Umkaschiervorrichtungen zwischen Foliendekaschierstelle und Aufkaschiereinrichtung vorzugsweise eine Entlastungseinrichtung auf, die zur zugentlasteten Führung der zugempfindlichen Folie auf zumindest einem Teilabschnitt der Folienführung von der Foliendekaschierstelle zur Aufkaschiereinrichtung ausgebildet ist.

Weitere Merkmale der Erfindung ergeben sich aus der nachfolgenden Beschreibung von Ausführungsbeispielen in Verbindung mit den Ansprüchen sowie den Figuren. In den Figuren werden gleiche bzw. ähnliche Bezugszeichen für funktionell gleichwertige oder ähnliche Charakteristiken unabhängig von speziellen Ausführungsformen verwendet. Es wird darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung von Ausführungsbeispielen der Erfindung wird auf die beiliegenden Figuren Bezug genommen, von denen
- Figur 1: die zum Verständnis wesentlichen Elemente einer ersten Ausführungsform einer erfindungsgemäßen Umkaschiervorrichtung in einer schematischen Darstellung zeigt und
- Figur 2: die zum Verständnis wesentlichen Elemente einer zweiten Ausführungsform einer erfindungsgemäßen Umkaschiervorrichtung in einer schematischen Darstellung zeigt.

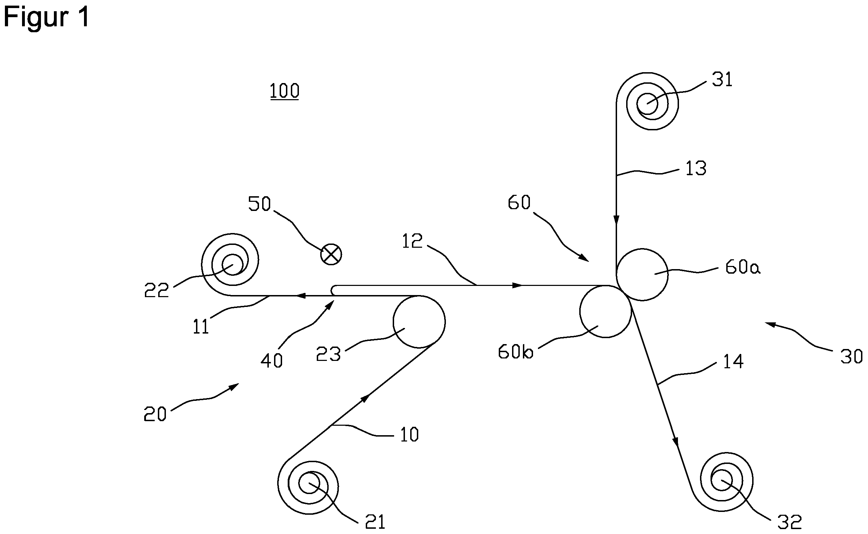

Die stark schematisierte Illustrationen von Figur 1 veranschaulicht den prinzipiellen Aufbau einer Vorrichtung 100 zum Umkaschieren zugempfindlicher Folien 12 von einem ersten bandförmigen Träger 11 auf einen zweiten bandförmigen Träger 13 gemäß einem ersten Ausführungsbeispiel.

Die Vorrichtung 100 weist eine erste Transporteinrichtung 20 und eine zweite Transporteinrichtung 30 auf. In dem dargestellten Ausführungsbeispiel umfasst die erste Transporteinrichtung 20 eine Laminatspenderrolle 21, eine Trägeraufwicklungsrolle 22 und eine Umlenkrolle 23. Die zweite Transporteinrichtung 30 umfasst im dargestellten Ausführungsbeispiel eine Trägerspenderrolle 31, eine Laminataufwicklungsrolle 32 und eine Aufkaschiereinrichtung 60.

Die Laminatspenderrolle 21 der ersten Transportvorrichtung 20 dient zur Abgabe eines Laminatbandes 10, wobei das Laminatband 10 üblicherweise nicht direkt auf die Spenderrolle 21, sondern auf einen (in der Figur nicht gezeigten) Spulenkörper aufgewickelt ist, der zum Abwickeln des Laminatbandes auf die Laminatspenderrolle 21 gesteckt wird. Die Laminatspenderrolle 21 ist wesentlicher Teil einer (in der Figur nicht näher dargestellten) Laminatausgabeeinrichtung, die als weitere Komponenten unter anderem auch eine Halterung und gegebenenfalls auch einen Antrieb zum Drehen der Laminatspenderrolle 21 umfasst.

Die Trägeraufwicklungsrolle 22 der ersten Transportvorrichtung 20 dient zur Aufnahme eines durch das Auftrennen des Laminatbandes 10 erhaltenen ersten Trägerbandes 11, wobei das Trägerband in gleicher Weise wie das Laminatband 10 üblicherweise nicht direkt auf die Rolle 22, sondern auf einen von der Trägeraufwicklungsrolle 22 gehaltenen (in der Figur nicht dargestellten) Spulenkörper aufgewickelt wird. Die Trägeraufwicklungsrolle 22 ist Kernbestandteil einer (in der Figur nicht näher dargestellten) Trägerbandaufnahmeeinrichtung, die als weitere Komponenten unter anderem auch eine Halterung sowie einen Antrieb zum Drehen der Trägeraufwicklungsrolle 22 umfasst.

Die Umlenkrolle 23 der ersten Transportvorrichtung 20 dient dem Umlenken eines von der Laminatausgabeeinrichtung ausgegebenen Laminatbandes 10 in Richtung der Dekaschierstelle 40, an der das Laminatband 10 in die zugempfindliche Folie 12 und den ersten bandförmigen Träger 11 aufgetrennt wird. Auch wenn in Figur 1 nur eine Umlenkrolle 23 dargestellt ist, können konkrete Ausgestaltungen der in Figur 1 schematisch dargestellten Umkaschiervorrichtung 100 auch eine mehrere Umlenkrollen 23 umfassende erste Transporteinrichtung 20 aufweisen. Ebenso können andere konkrete Ausgestaltungen der Umkaschiervorrichtung 100 eine Transporteinrichtung 20 aufweisen, bei der das Laminatband 10 bzw. das nach dem Ablösen der zugempfindlichen Folie 12 verbleibende Trägerband 11 direkt von der Laminatausgabeeinrichtung zur Trägerbandaufnahmeeinrichtung, d. h. ohne Umweg über eine Umlenkrolle 23, geführt wird.

Die Trägerspenderrolle 31 der zweiten Transportvorrichtung 30 dient der Ausgabe eines aufgewickelten zweiten Trägerbandes 13, auf das die zugempfindliche Folie 12 aufkaschiert werden soll. Wie das Laminatband 10 auf der Laminatspenderrolle 21 ist auch das zweite Trägerband 13 üblicherweise nicht direkt auf die Trägerspenderrolle 31, sondern auf einen (in der Figur nicht gezeigten) Spulenkörper aufgewickelt, der zum Abwickeln des zweiten Trägerbandes 13 auf die Trägerspenderrolle 31 gesteckt wird. Die Trägerspenderrolle 31 ist Teil einer (in der Figur nicht näher dargestellten) Trägerausgabeeinrichtung, die als weitere Komponenten unter anderem auch eine Halterung und gegebenenfalls auch einen Antrieb für die Trägerspenderrolle 31 umfasst.

Die Laminataufwicklungsrolle 32 der zweiten Transportvorrichtung 30 dient der Aufnahme eines zweiten Laminatbandes 14, das durch Aufkaschieren der von einem Laminatband 10 dekaschierten zugempfindlichen Folie 12 auf das zweite Trägerband 13 erhalten wird. Wie das erste Trägerband 11 bei der Trägeraufwicklungsrolle 22 wird auch das zweite Laminatband 14 üblicherweise nicht direkt auf die Rolle 32, sondern auf einen von der Laminataufwicklungsrolle 32 gehaltenen (in der Figur nicht dargestellten) Spulenkörper aufgewickelt. Die Laminataufwicklungsrolle 32 ist Kernbestandteil einer (in der Figur nicht näher dargestellten) Laminatbandaufnahmeeinrichtung, die als weitere Komponenten unter anderem auch eine Halterung und einen Antrieb für die Laminataufwicklungsrolle 32 umfasst.

Die Aufkaschiereinrichtung 60 der zweiten Transporteinrichtung 30 dient neben dem Transport von zugempfindlicher Folie 12, zweitem Trägerband 13 und zweitem Laminatband 14 vor allem dem Aufkaschieren der zugempfindlichen Folie 12 auf den zweiten bandförmigen Träger 13. In der in Figur 1 dargestellten Ausführungsform weist die Aufkaschiereinrichtung 60 als wesentliche Elemente zwei Walzen 60a und 60b auf, zwischen denen ein Nip ausgebildet ist. Unter Nip ist in dieser Schrift und in Übereinstimmung mit dem üblichen Sprachgebrauch der Bereich zwischen zwei Walzen zu verstehen, in dem diese entweder direkt oder vermittelt über ein sich zwischen den Walzen befindliches Medium, im vorliegenden Fall ein zweites Trägerband 13 mit darauf befindlicher zugempfindlicher Folie, aufeinander einwirken bzw. Kräfte aufeinander ausüben. Die Existenz eines Nips setzt voraus, dass die Walzen entweder sich oder ein dazwischen befindliches Medium berühren. Das zweite Trägerband 13 wird dem Nip über einen Teilumfang der ersten Walze 60a zugeführt, das zuvor von dem ersten Laminatband abgetrennte zugempfindliche Folienband 12 wird dem Nip dagegen über einen Teilumfang der zweiten Walze 60b zugeführt. Das Aufkaschieren der Folie 12 auf den Träger 13 erfolgt im Nip mit einem Anpressdruck, der über die von den beiden Walzen 60a und 60b aufeinander ausgeübten Andruckkräfte vermittelt wird.

Als für die bandförmigen Träger 11 bzw. 13 geeignete Materialien können beispielsweise Polyester, Polypropylen, Polyvinylchlorid, Aluminium und Papier genannt werden, wobei zumindest eine Seite des Trägerbandes gegebenenfalls eine Siliconbeschichtung, Polyethylenbeschichtung, Fluorsiliconbeschichtung oder Polytetrafluorethylenbeschichtung aufweist. Die Trägerbänder weisen je nach Anwendungsfall üblicherweise eine Dicke von etwa 6 bis etwa 200 µm auf.

Die zugempfindliche Folie 12 weist üblicherweise eine Dicke aus dem Bereich von etwa 2 bis etwa 15 µm auf und kann auf einem Polymer basieren, das aus der Gruppe bestehend aus Polyolefinen, Olefin-Copolymerisaten, Polyestern, Co-Polyestern, Polyamiden, CoPolyamiden, Polyurethanen, und dergleichen mehr ausgewählt ist. Als Beispiele für geeignete Materialien können Polyester und hiervon insbesondere Polyethylenterephthalate als auch Polycarbonate genannt werden, Polyolefine wie z. B. Polyethylene, Polypropylene, Polybutylene oder Polyisobutylene, Polyethylenoxide, Polyurethane, Polystyrole, Polyamide, Polyimide, Polyvinylacetate, Polyvinylchloride, Polyvinylidenchloride, Copolymerisate wie beispielsweise AcrylnitrilButadien-Styrol-Terpolymere oder Ethylen-VinylacetatCopolymerisate.

Die zugempfindliche Folie 12 kann zudem eine haftklebende Polymerschicht aufweisen, die auf einem wie zuvor beschriebenen Folienmaterial aufgebracht ist. Die genaue Zusammensetzung des Matrixmaterials richtet sich nach dem jeweiligen Anwendungszweck, bei der Herstellung von transdermalen therapeutischen Systemen z. B. nach dem bzw. den zu verabreichenden Wirkstoff(en) und eventuell hierfür weiter erforderlichen Stoffen, wie zum Beispiel Permeationsförderern. Als zur Ausbildung einer Polymermatrix geeignete Materialien sind beispielsweise Homo- und Copolymere von (Meth)acrylaten, Polyvinylether, Polyisobutylene, Polyisoprenkautschuk, Styrol-Butadien-Copolymere, Styrol-Butadien-Styrol-Copolymere bekannt. Von den (Meth)acrylat-Copolymeren können beispielsweise die Copolymere von Alkylacrylaten und/oder Alkylmethacrylaten und weiteren ungesättigten Monomeren genannt werden, wie Acrylsäure, Methacrylsäure, Acrylamid, Dimethylacrylamind, Dimethylaminoethylacrylamid, Acrylnitril und/oder Vinylacetat.

Beim in Figur 1 veranschaulichten bestimmungsgemäßen Einsatz der Vorrichtung 100 ist das von der Laminatausgabeeinrichtung abgegebene Laminatband 10 über eine Umlenkrolle 23 zu einer Foliendekaschierstelle 40 geführt. Zur besseren Veranschaulichung sind die bestimmungsgemäßen Laufrichtungen der einzelnen Bänder in den Figuren durch Pfeile gekennzeichnet. Unter dem Begriff der "Foliendekaschierstelle" ist in dieser Schrift eine Stelle zu verstehen, an der das Folienband 12 von dem Laminatband 10 abgezogen, bzw. an der das Laminatband 10 zum Ablösen des Folienbandes aufgetrennt wird. Der nach dem Dekaschieren der zugempfindlichen Folie 12 verbleibende Teil des Laminatbandes 10, d. h. das erste Trägerband 11, wird von der Foliendekaschierstelle 40 weiter zur Trägerbandaufnahmeeinrichtung 22 geführt, wo es auf der Trägeraufwicklungsrolle 22 bzw. auf einen darauf befindlichen Spulenkörper aufgewickelt wird. An der Foliendekaschierstelle 40 erfährt die zugempfindliche Folie bzw. das zugempfindliche Folienband 12 eine Umlenkung in eine zur ursprünglichen Laufrichtung des Laminats 10 an der Foliendekaschierstelle 40 entgegengesetzte Richtung. Im Idealfall schließen ursprüngliche und entgegengesetzte Richtung einen Winkel von 180° ein, so dass der Biegeradius der zugempfindlichen Folie an der Foliendekaschierstelle 40 minimal ist.

Die Ablösbarkeit der zugempfindlichen Folie 12 wird durch die Trennkraft bestimmt, die zum Abziehen der Folie 12 vom Träger 11 an der sich an der Foliendekaschierstelle 40 bildenden, in etwa linienförmigen Trennstelle erforderlich ist. Wesentlich ist hierbei die Kraftkomponente, die am Übergang von bereits abgelöster und noch am Träger 11 haftender Folie 12 senkrecht zur Grenzfläche zwischen diesen beiden Komponenten des ersten Laminats 10 orientiert ist. Je kleiner der Biegeradius der Folie 12 an der Foliendekaschierstelle 40 ist, desto größer ist der Anteil dieser Kraftkomponente an der insgesamt auf den Übergang einwirkenden Trennkraft. Ferner hat sich gezeigt, dass sich bei kleinen Biegeradien die Trennkraft auf die Trennstelle, d. h. den Übergang von bereits abgelöster und noch am Träger 11 haftender Folie 12 konzentriert. Da die Trennkraft durch die auf die zugempfindliche Folie 12 ausgeübte Zugkraft aufgebracht wird, kann somit ein Auftrennen des ersten Laminatbandes 10 mit geringen Zugkräften und in der Folge mit geringen Dehnungen des Folienbandes 12 erreicht werden.

Um die hierfür erforderlichen kleinen Biegeradien sicherzustellen, erfolgt das Abziehen der Folie 12 wie in Figur 1 veranschaulicht in Form einer freien Schlaufe, d.h. ohne Verwendung einer Umlenkrolle oder Spenderkante an der Foliendekaschierstelle 40. Auch wenn die Umsetzung der auf die Folie 12 ausgeübten Zugkraft in eine effektive Trennkraft bei eine Umlenkung der Folie 12 an der freien Schlaufe maximal ist, bei der die Richtungsänderung der zugempfindlichen Folie 180° beträgt, nimmt die Trennkraft bei gleichen Zugkräften nur unwesentlich ab, wenn die Umlenkung des Folienbandes an der Dekaschierstelle 40 nur etwa 150° beträgt. Vorteilhaft weist die Richtungsänderung der Folie an der Dekaschierstelle daher einen Wert aus dem Bereich von 150° bis 180° auf, wobei die Richtungsänderung, z. B. wenn die Foliendekaschierstelle 40 direkt auf der Umlenkrolle 23 angeordnet ist, auch etwas mehr als 180° betragen kann.

Um das Ablösen des zugempfindlichen Folienbandes 12 von dem Trägerband 11 möglichst gleichmäßig zu gestalten und Schwankungen der auf die Folie 12 einwirkenden Zugkräfte somit möglichst gering zu halten, erfolgt die Steuerung von erster und zweiter Transporteinrichtung vorzugsweise als Funktion der Lage der Foliendekaschierstelle 40 und zwar so, dass die Foliendekaschierstelle praktisch, d.h. innerhalb der technischen Regelgenauigkeit, nicht wandert. Um eine solche stationäre Lage der Foliendekaschierstelle 40 zu gewährleisten, wird ein die Lage der freien Schlaufe erfassender Sensor 50 verwendet. Da der Biegeradius der beim Abziehen der Folie 12 gebildeten freien Schlaufe sehr klein ist, kann als Sensor jede Einrichtung verwendet werden, mit deren Hilfe eine Lagebestimmung einer Kante ermöglicht wird. Als Beispiele für geeignete Sensoren können daher auf Lichtstreuung basierende Sensorsysteme sowie bildverarbeitende Einrichtungen genannt werden, wobei auch andere Sensorsysteme verwendet werden können, beispielsweise solche, die auf einem unterschiedlichen Reflexionsverhalten von abgezogener Folienseite und freigelegter Trägerbandseite beruhen. Der Sensor 50 bzw. das Sensorsystem 50 geben vorzugsweise ein Sensorsignal aus, dessen Wert repräsentativ für die jeweils aktuelle Lage der Foliendekaschierstelle 40 ist und von der (in den Figuren nicht gezeigten) Steuerung zur Stabilisierung der stationären Lage der Foliendekaschierstelle 40 verwendet wird.

Die Steuerung der stationären Lage der Foliendekaschierstelle 40 erfolgt im Wesentlichen über eine Abstimmung der Transportgeschwindigkeiten von erstem Laminat 10 und zweitem Laminat 14, da hierüber die auf das von dem ersten Laminatband 10 dekaschierte Folienband 12 ausgeübten Zugkräfte bestimmt werden. Bei Ausgestaltungen der Vorrichtung 100 werden hierzu lediglich die Trägeraufwicklungsrolle 11 sowie die Laminataufwicklungsrolle 32 aktiv angetrieben. Laminat- und Trägerspenderrolle 21 bzw. 31 werden jeweils passiv über das Laminatband 10 bzw. das Trägerband 13 angetrieben. Dies gilt bei Ausgestaltungen auch für die Umlenkrolle 23 und die beiden Walzen 60a und 60b der Aufkaschiereinrichtung 60. Bei anderen Ausgestaltungen einer Umkaschiervorrichtung 100 erfolgt die Lagesteuerung der Foliendekaschierstelle 40 mittels Abstimmung der Antriebe von Trägeraufwicklungsrolle 22 und Aufkaschiereinrichtung 60, wobei in der Regel nur eine der beiden Walzen 60a oder 60b aktiv angetrieben wird. Die Abstimmung der Antriebe erfolgt wie zuvor in Abhängigkeit der mittels des Sensors 50 jeweils aktuell bestimmten Lage der freien Schlaufe oder auch abhängig von einer Lageänderungsgeschwindigkeit der Schlaufe, d. h. auf der Basis eines entsprechenden Sensorsignals als Regelgröße.

Das Abziehen der zugempfindlichen Folie 12 in einer wie zuvor beschriebenen freien Schlaufe minimiert die beim Abziehen auf die Folie einwirkenden Zugkräfte. Die Lagesteuerung der Foliendekaschierstelle 40 stellt darüber hinaus sicher, dass die durch die Zugkräfte aufgebrachten Trennkräfte stets an die jeweils aktuelle Klebkraft bzw. Adhäsionskraft angepasst sind und somit keine Sicherheitsreserven umfassenden Zugkräfte auf das Folienband ausgeübt werden müssen.

Die in Figur 1 veranschaulichte Umkaschiervorrichtung 100 eignet sich insbesondere für zugempfindliche Folienbänder 12, die von einem dünnen zugempfindlichen Kunststoffband gebildet sind, das an der zum Trägerband 11 weisenden Seite mit einer haftklebenden Schicht, beispielsweise eine selbstklebenden wirkstoffhaltigen Polymerschicht, beschichtet ist. Selbstverständlich eignet sich die Vorrichtung 100 ferner auch zum Umkaschieren von Folienbändern 40, die ohne Kleber direkt auf dem Trägerband 11 haften, beispielsweise durch vorhergehendes Aufkaschieren unter Druck und/oder erhöhten Temperaturen.

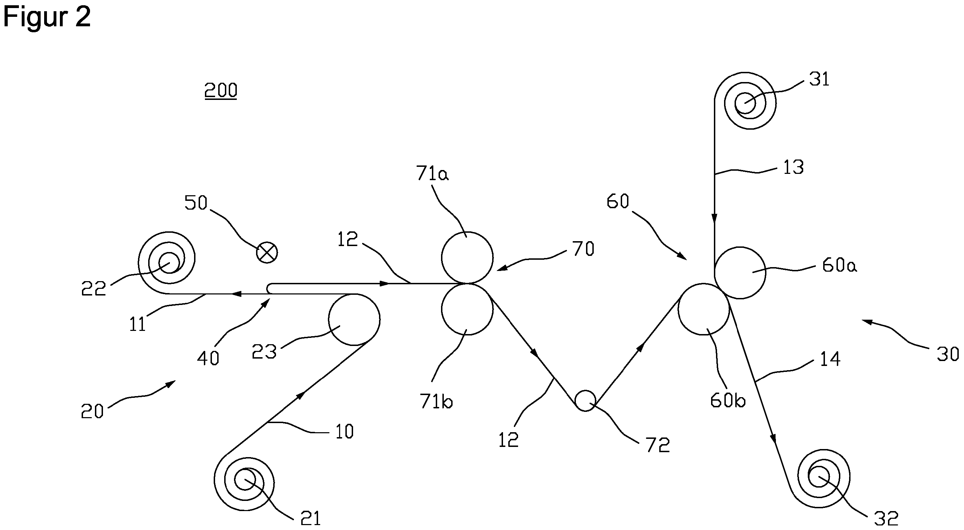

Im Unterschied zu der in Figur 1 illustrierten Umkaschiervorrichtung 100 weist die in der schematischen Darstellung von Figur 2 veranschaulichte Umkaschiervorrichtung 200 eine Zugentlastungseinrichtung 70 auf, die zwischen der Foliendekaschierstelle 40 und der Aufkaschiereinrichtung 60 angeordnet ist. Mit Hilfe der Zugentlastungseinrichtung 70 lassen sich eventuelle Dehnungen der zugempfindlichen Folie 12 vor dem Aufkaschieren rückgängig machen oder zumindest auf ein unschädliches Maß reduzieren. Da die übrigen Komponenten der Umkaschiervorrichtung 200 denen der Umkaschiervorrichtung 100 entsprechen, wird im Folgenden lediglich auf die Zugentlastungseinrichtung 70 eingegangen und im Übrigen auf die vorherigen Ausführungen verwiesen.

Die in Figur 2 veranschaulichte Zugentlastungseinrichtung weist einen die Walzen 71a und 71b umfassenden Antrieb 71 und eine sogenannte Tänzerwalze 72 auf. Wie bei der zuvor beschriebenen Umkaschiervorrichtung 100 wird das erste Laminatband 10 gegebenenfalls über eine optionale Umlenkrolle 23 zunächst der Foliendekaschierstelle 40 zugeführt. Zum Ablösen wird das an der Foliendekaschierstelle 40 entgegen der ursprünglichen Transportrichtung des Laminatbandes 10 in einer freien Schlaufe umgelenkte zugempfindliche Folienband 12 durch den zwischen den beiden Antriebswalzen 71a und 71b gebildeten Nip geführt, in dem die zum Ablösen erforderlichen Zugkräfte auf das Folienband 12 ausgeübt werden.

Im Unterschied zu einer Umkaschiervorrichtung 100 gemäß der Darstellung von Figur 1 erfolgt die Steuerung der stationären Lage der Foliendekaschierstelle 40 bei einer wie in Figur 2 veranschaulichten Umkaschiervorrichtung 200 vorzugsweise über eine Steuerung von erster Transporteinrichtung 20 und Antrieb 71. Die Steuerung bewirkt hierbei insbesondere eine Abstimmung der Transportgeschwindigkeiten von erstem Laminat 10 und dekaschiertem Folienband 12 so, dass die Lage der freien Schlaufe innerhalb der technischen Regelgenauigkeit ortsfest bleibt.

Der in Figur 2 veranschaulichte Walzenpaar-Antrieb 71 eignet sich insbesondere zum Dekaschieren von zugempfindlichen Folien, die keine haftklebende Oberfläche aufweisen. Alternative Ausgestaltungen einer Umkaschiervorrichtung 200 weisen statt dem Walzenpaar einen Antrieb auf Basis einer Vakuumsaugwalze oder eines Vakuumsaugbandes auf, die bzw. das zum Transport des Folienbands an dessen nichtklebender Oberfläche anliegt.

Nach dem Antrieb 71 durchläuft das Folienband 12 eine Beruhigungsstrecke, in der keine oder nur sehr geringe Zugkräfte auf die Folie 12 ausgeübt werden, wodurch sich Dehnungen der Folie zurückbilden können. Zur Ausbildung der Beruhigungsstrecke der Zugentlastungseinrichtung 70 wird das Folienband 12 zwischen Antrieb 71 und Aufkaschiereinrichtung 60 vorzugsweise durchhängend geführt. Gegebenenfalls kann der Durchhang wie in Figur 2 dargestellt mittels einer Tänzerwalze 72, d. h. einer lose in die durchhängende Folie 12 eingelegten Walze geringen Gewichts, stabilisiert werden. Die Lage der Tänzerwalze 72 (bzw. bei einem freien Durchhängen des Folienbands dessen Durchhang) kann bei Ausgestaltungen der Umkaschiervorrichtung 200 mittels eines oder mehrerer (in der Figur nicht gezeigter) Sensoren überwacht und die Geschwindigkeit der Aufkaschiereinrichtung 60 abhängig von den von diesen Sensoren erhaltenen Signalen so gesteuert werden, dass eine stationäre Lage der Tänzerwalze 72 (bzw. des Foliendurchhangs) erzielt wird.

Die in Figur 2 dargestellte Umkaschiervorrichtung 200 eignet sich insbesondere zum Umkaschieren von Folienbändern 12, die beim Ablösen von dem ersten Trägerband 11 eine nicht tolerierbare Dehnung erfahren.

## Patentansprüche

1. Vorrichtung zum Umkaschieren zugempfindlicher Folien (12) von einem ersten Träger (11) auf einen zweiten Träger (13), wobei die Vorrichtung (100, 200) eine erste Transporteinrichtung (20), eine zweite Transporteinrichtung (30) und eine Aufkaschiereinrichtung (60) aufweist und wobei die
- erste Transporteinrichtung (20) zum Transport eines ersten Laminatbandes (10), das von einem ersten bandförmigen Träger (11) und einer darauf aufkaschierten zugempfindlichen Folie (12) gebildet ist, von einer Laminatausgabeeinrichtung zu einer Foliendekaschierstelle (40) und zum Weitertransport des an der Foliendekaschierstelle (40) von der zugempfindlichen Folie (12) abgetrennten Trägerbandes (11) von der Foliendekaschierstelle (40) zu einer Trägerbandaufnahmeeinrichtung ausgebildet ist,
- die zweite Transportvorrichtung (30) zum Transport eines zweiten bandförmigen Trägers (13) zur Aufkaschiereinrichtung (60), die zum Aufkaschieren einer an der Foliendekaschierstelle (40) dekaschierten zugempfindlichen Folie (12) auf den zweiten Träger (13) zur Bildung eines zweiten Laminatbandes (14) ausgebildet ist, und zum Weitertransport des zweiten Laminatbandes von der Aufkaschiereinrichtung (60) zu einer Laminatbandaufnahmeeinrichtung ausgebildet ist, **gekennzeichnet dadurch, dass**
- die erste Transportvorrichtung (20) und die Aufkaschiereinrichtung (60) relativ zueinander so angeordnet sind, dass die zugempfindliche Folie (12) an der Foliendekaschierstelle (40) in zur Laufrichtung des ersten Laminatbandes (10) an dieser Stelle entgegengesetzten Richtung abgezogen wird.

2. Vorrichtung nach Anspruch 1, die ferner eine Einrichtung zur Lagestabilisierung der Foliendekaschierstelle umfasst.

3. Vorrichtung nach Anspruch 2, worin die Einrichtung zur Lagestabilisierung der Foliendekaschierstelle zumindest einen Sensor aufweist, der zur Ausgabe von zumindest einem Sensorsignal ausgebildet ist, das repräsentativ für eine aktuelle Lage der Foliendekaschierstelle ist.

4. Vorrichtung nach Anspruch 3, die ferner eine Steuerung aufweist, die so zur Steuerung von erster Transportvorrichtung und zweiter Transportvorrichtung in Abhängigkeit des Sensorsignals ausgebildet ist, dass sich die Lage der Foliendekaschierstelle relativ zu erster Transportvorrichtung und Aufkaschiereinrichtung nicht verändert.

5. Vorrichtung nach einem der Ansprüche 1 bis 4, die zur direkten Führung der dekaschierten zugempfindlichen Folie von der Foliendekaschierstelle zur Aufkaschiereinrichtung ausgebildet ist.

6. Vorrichtung nach einem der Ansprüche 1 bis 4, die ferner eine Entlastungseinrichtung aufweist, die zur zugentlasteten Führung der zugempfindlichen Folie auf zumindest einem Teilabschnitt der Folienführung zwischen Foliendekaschierstelle und Aufkaschiereinrichtung ausgebildet ist.

## Claims

1. An apparatus for translaminating tension-sensitive films (12) from a first liner (11) to a second liner (13), the apparatus (100, 200) having a first transport device (20), a second transport device (30), and a laminating device (60), wherein
- the first transport device (20) is configured to transport a first laminate strip (10) formed by a first strip-like liner (11) and a tension-sensitive film (12) laminated thereon from a laminate supply device to a film delaminating position (40), and to further transport the liner strip (11) separated from the tension-sensitive film (12) at the film delaminating position (40) from the film delaminating position (40) to a liner strip receiving device,
- the second transport device (30) is configured to transport a second strip-like liner (13) to the laminating device (60) configured for laminating a tension-sensitive film (12) delaminated at the film delaminating position (40) onto the second liner (13) for forming a second laminate strip (14), and to further transport the second laminate strip from the laminating device (60) to a laminate strip receiving device, **characterized in that**
- the first transport device (20) and the laminating device (60) are arranged with respect to each other such that the tension-sensitive film (12) is peeled off at the film delaminating position (40) in the direction opposite to the direction of movement of the first laminate strip (10) at this position.

2. The apparatus of claim 1, further comprising a device for stabilizing the position of the film delaminating position.

3. The apparatus of claim 2, wherein the device for stabilizing the position of the film delaminating position comprises at least one sensor configured to output at least one sensor signal representative of a current position of the film delaminating position.

4. The apparatus of claim 3, further comprising a controller configured to control the first transport device and the second transport device based on the sensor signal such that the position of the film delaminating position does not change with respect to the first transport device and the laminating device.

5. The apparatus of one of claims 1 to 4, configured for directly guiding the delaminated tension-sensitive film from the delaminating position to the laminating device.

6. The apparatus of one of claims 1 to 4, further comprising a tension relief device configured for a tension-relieved guiding of the tension-sensitive film over at least a part of the film guidance between the film delaminating position and the laminating device.

## Revendications

1. Dispositif de translation de feuilles sensibles a la traction (12) d'un premier support (11) sur un deuxième support (13), dans lequel le dispositif (100, 200) présente un premier dispositif de transport (20), un deuxième dispositif de transport (30) et un dispositif de contrecollage (60), et dans lequel
- le premier dispositif de transport (20) est configure pour le transport d'une première bande stratifiée (10), qui est formée d'un premier support en forme de bande (11) et d'une feuille sensible a la traction (12) contrecollée sur celui-ci, d'un dispositif de alimentation de stratifiés à un poste de décollement de feuille (40) et pour le transfert de la bande de support (11), séparée de la feuille sensible à la traction (12) au poste de décollement de feuille (40), du poste de décollement de feuille (40) à un dispositif de réception de bande de support,
- le deuxième dispositif de transport (30) est configuré pour le transport d'un deuxième support en forme de bande (13) vers le dispositif de contrecollage (60), qui est configuré pour le contrecollage d'une feuille sensible à la traction (12) décollée au poste de décollement de feuille (40), sur le deuxième support (13) pour former une deuxième bande stratifiée (14), et pour le transfert de la deuxième bande stratifiée du dispositif de contrecollage (60) à un dispositif de réception de bande stratifiée, **caractérisé en ce que**
- le premier dispositif de transport (20) et le dispositif de contrecollage (60) sont disposés l'un par rapport à l'autre, de telle manière que la feuille sensible a la traction (12) soit retirée au poste de décollement de feuille (40) dans une direction opposée à la direction de progression de la première bande stratifiée (10) à cet endroit.

2. Dispositif selon la revendication 1, qui comprend en outre un dispositif pour stabiliser la position du poste de décollement de feuille.

3. Dispositif selon la revendication 2, dans lequel le dispositif pour stabiliser la position du poste de décollement de feuille présente au moins un capteur, qui est conçu de façon à émettre au moins un signal de capteur, qui est représentatif d'une position actuelle du poste de décollement de feuille.

4. Dispositif selon la revendication 3, qui présente en outre une commande, qui est configurée pour la commande du premier dispositif de transport et du deuxième dispositif de transport en fonction du signal de capteur, de telle manière que la position du poste de décollement de feuille par rapport au premier dispositif de transport et au dispositif de contrecollage ne change pas.

5. Dispositif selon l'une quelconque des revendications 1 à 4, qui est configure pour le guidage direct de la feuille sensible a la traction décollée du poste de décollement de feuille au dispositif de contrecollage.

6. Dispositif selon l'une quelconque des revendications 1 à 4, qui présente en outre un dispositif de détente, qui est configure pour le guidage détendu en traction de la feuille sensible a la traction sur au moins une section partielle du guidage de feuille entre le poste de décollement de feuille et le dispositif de contrecollage.
